# EUROPÄISCHE PATENTSCHRIFT

(11) **EP 2 323 577 B1**
(45) Veröffentlichungstag und Bekanntmachung des Hinweises auf die Patenterteilung: **20.04.2016**
(21) Anmeldenummer: 09777871.6
(22) Anmeldetag: 13.08.2009
(51) Int. Cl.: A61B 18/12

(54) **SOFTGENERATOR**
SOFT GENERATOR
GÉNÉRATEUR SOUPLE

(30) Priorität: 08.09.2008 DE 102008046248; 04.11.2008 DE 102008055820
(43) Veröffentlichungstag der Anmeldung: 25.05.2011
(73) Patentinhaber: ERBE Elektromedizin GmbH, 72072 Tübingen (DE)
(72) Erfinder: SCHALL, Heiko, 72622 Nürtingen (DE); EISELE, Florian, 79108 Freiburg (DE)
(74) Vertreter: Bohnenberger, Johannes
(86) Internationale Anmeldenummer: PCT/EP2009/005889
(87) Internationale Veröffentlichungsnummer: WO 2010/025815

(56) Entgegenhaltungen:
- EP-A2- 1 693 015
- WO-A1-96/39085
- WO-A1-98/07378
- WO-A1-98/47436
- DE-A1- 3 510 586
- DE-A1- 3 708 801
- US-A- 5 442 539
- US-A1- 2002 022 836
- US-A1- 2005 099 827
- US-A1- 2007 118 102
- US-A1- 2008 082 096
- US-B1- 6 565 558

## Beschreibung

Die Erfindung betrifft einen HF-Chirurgiegenerator zur Erzeugung von Ausgangsleistungen mit hohem Wirkungsgrad gemäß dem Oberbegriff des Anspruchs 1 mit einer ersten Stufe zur Erzeugung einer HF-Leistung, die mit einer zweiten Stufe gekoppelt ist, wobei die zweite Stufe einen Eingang (A, B) und einen Ausgang (C, D) aufweist, und wobei sich zwischen dem Eingang (A, B) und dem Ausgang (C, D) der zweiten Stufe ein Serienresonanz-Schaltkreis befindet.

Die stetige Entwicklung der HF-Chirurgie in den letzten Jahren führte zu Verfahren zur Kontaktkoagulation (Tumordevitalisierung), zu Methoden für (Unterwasser-) Gewebeschnitte und für (Unterwasser-) Gewebevaporisation. Zur Durchführung dieser Verfahren werden HF-Generatoren eingesetzt, die mit hohen Dauerleistungen und mit sehr hohen Pulsleistungen und/oder langen Aktivierungszeiten arbeiten. Zugleich steigen jedoch auch die Anforderungen an die elektromagnetische Verträglichkeit (EMV) der eingesetzten HF-Generatoren, weil die Störung anderer elektromedizinischer Geräte, beispielsweise für das Patientenmonitoring oder zur Diagnostik, immer weniger akzeptabel wird. Die notwendigen Maßnahmen zur Gewährleistung der Eigenstörfestigkeit solcher HF-Generatoren, die trotzdem die geforderten hohen HF-Leistungen erzielen, sind dadurch nur noch unter einem hohem entwicklungstechnischen Aufwand zu realisieren.

Aus dem Stand der Technik ist es bekannt, zur HF-Leistungserzeugung Schaltungsanordnungen bestehend aus Serien- und/oder Parallelresonanzkreisen zu verwenden, die von Leistungshalbleitern im Schaltbetrieb gespeist werden. Für die Ausgangscharakteristik der HF-Generatoren sind neben den Netzteileigenschaften (inklusive ggf. vorhandener Regelungen) vor allem die Gestaltung dieser Resonanzkreise und die Art ihrer Speisung - die ebenso als Stellgröße für Regelungen dienen kann - von entscheidender Bedeutung. Dabei ist bekannt, dass die Resonanzfrequenz sowie der Eingangswiderstand und mithin das Übersetzungsverhältnis solcher Resonanzkreise im Wesentlichen von dem Lastwiderstand abhängig sind. Dies führt bei den aus dem Stand der Technik bekannten Schaltungsanordnungen zur Realisierung von HF-Generatoren mit Resonanzkreisen dazu, dass sich je nach Betriebsart bei einer bestimmten Frequenz die Resonanz aufspaltet, da die im HF-Generator verwendeten Serien- und Parallelresonanzkreise ab einem bestimmten Lastwiderstandsbereich miteinander interagieren. Um dennoch die geforderte hohe Ausgangsleistung zu erzielen, wären jedoch unter anderem ungünstig hohe Netzteilströme notwendig. Dies wirkt sich einerseits negativ auf den Wirkungsgrad des HF-Generators aus und führt andererseits zu einem Betrieb des HF-Generators mit nicht-sinusförmigen Eingangsstrom, wodurch sich auch die EMV-Eigenschaften des HF-Generators verschlechtern.

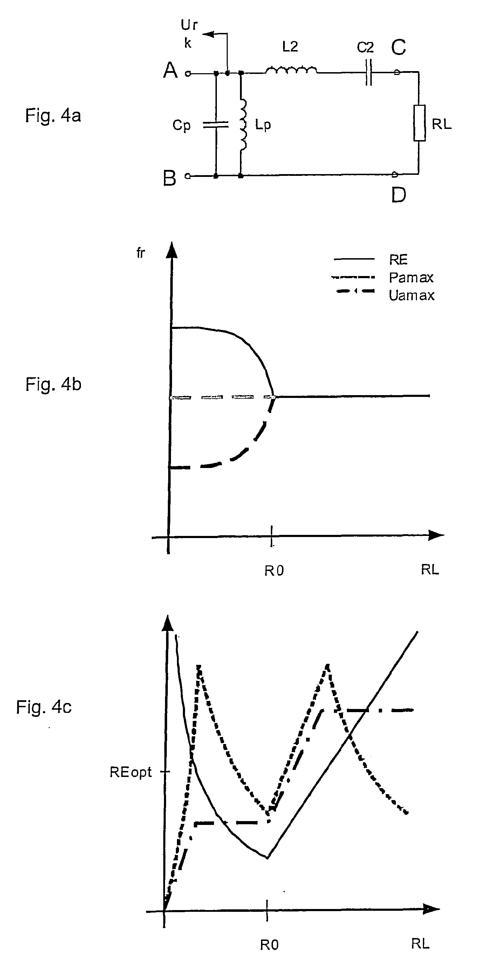

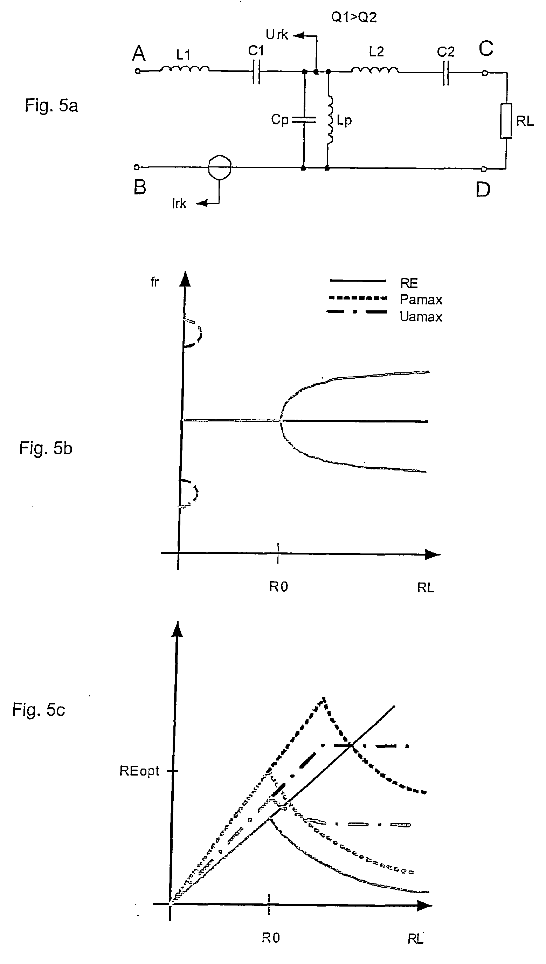

Die Fig. 4a und Fig. 5a zeigen verschiedene aus dem Stand der Technik bekannte Topologien von Resonanzkreisen, wie sie zur Erzeugung von HF-Leistungen eingesetzt werden. In diesen Fällen lässt sich für eine zusätzliche lastwiderstandsunabhängige Impedanztransformation durch die Struktur Lp, L₂ ein (streuungsbehafteter) Transformator integrieren.

Die Fig. 4b, c und Fig. 5b,c zeigen die zur jeweiligen Topologie gehörigen Verläufe der Resonanzfrequenz fᵣ, des Filtereingangswiderstandes RE, sowie der maximalen Ausgangsleistung P_{a,max} und - spannung U_{a,max} als Funktion des Lastwiderstandes RL. Die beiden letzteren sind zusätzlich von der Art der Speisung abhängig.

Hier wurde eine Speisung mit einer Rechteckspannung der entsprechenden Resonanzfrequenz und ein Netzteil mit einer maximalen Ausgangsspannung U₀ und einem maximalen Ausgangsstrom I₀ angenommen. Unter diesen Bedingungen transformiert sich der optimale Lastwiderstand Rₒₚₜ=U₀/I₀ in den optimalen Filtereingangswiderstand REopt=8/pi^2*Rₒₚₜ.

Für RE<REₒₚₜ arbeitet das Netzteil in der Strombegrenzung und für RE>REopt in der Spannungsbegrenzung. In Anlehnung an die Eigenschaften idealer Resonanzkreise werden Resonanzstellen, an welchen sich das Filter wie ein Serienresonanzkreis (SRK) bzw. Parallelresonanzkreis (PRK) verhält, als Serienresonanzen (SR) bzw. Parallelresonanzen (PR) bezeichnet. In den Fig. 4b,c und Fig. 5b,c sind Serienresonanzen mit unterbrochener Linie und Parallelresonanzen mit durchgezogener Linie dargestellt.

Darüber hinaus zeigen die Fig. 4b und 5b jeweils alle für das Filter möglichen Resonanzstellen, aber nur die bei der jeweils gewählten Art der Frequenzrückkopplung tatsächlich auftretenden Betriebsfrequenzen sind mit durchgehender Linie dargestellt. Nur für diese Frequenzen sind in den Fig. 4c und 5c der Filtereingangswiderstand RE mit durchgehender Linie sowie die maximale Ausgangsleistung P_{a,max} mit unterbrochener Linie und die maximale Ausgangsspitzenspannung Uₐₘₐₓₚ mit strichpunktierter Linie dargestellt.

Zur Charakterisierung der Kurvenverläufe sind die belasteten Güten der Einzelresonanzkreise Q1=1/RL*sqrt(L1/C1), Q2=1/RL*sqrt(L2/C2) und Qp=RL*sqrt(Cp/Lp) hilfreich.

Die Verzweigung der Resonanzkurven findet beim Lastwiderstand R₀ statt, der sich in Fig. 4b aus Qp=Q2 und in Fig. 5b aus Qp=Q₁+Q2 ergibt. Die Verzweigung findet, wie hier dargestellt, dann genau in einem Punkt statt, wenn die Resonanzfrequenzen der Einzelresonanzkreise übereinstimmen.

Die Fig. 4a zeigt einen aus dem Stand der Technik bekannten Resonanzkreis zur Erzeugung von Ausgangsleistungen mit einem am Eingang A, B liegenden Parallelresonanzschwingkreis (PRK) bestehend aus einer Kapazität Cₚ und einer Induktivität Lp, einem Serienresonanzschwingkreis mit einer Induktivität L₂ und Kapazität C₂ und einer Last R_{L} am Ausgang C, D. Diese Anordnung ist in Zusammenhang mit der gängigerweise angewandten Spannungsspeisung ungeeignet zur Erfüllung der Anforderungen nach hoher HF-Leistungsabgabe und gutem Wirkungsgrad, weil sich dabei ein stark nichtsinusförmiger Eingangsstrom einstellt. Zwar würde hier eine Stromspeisung entsprechende Abhilfe schaffen. Die Speisung mit einer Stromquelle ist jedoch vergleichsweise aufwändig.

Aus der Fig. 4b ist ersichtlich, dass sich die Resonanzfrequenz für kleine Lastwiderstände aufspaltet, weil der Parallelresonanzschwingkreis PRK und Ausgangs-Serienresonanzschwingkreis SRK interagieren. Dadurch kommt es zu einer Erhöhung der Resonanzfrequenz mit abnehmendem Lastwiderstand.

Fig. 5a zeigt einen aus dem Stand der Technik bekannten Resonanzkreis zur Erzeugung von Ausgangsleistungen mit einem am Eingang A, B liegenden Serienresonanzschwingkreis SRK bestehend aus einer Induktivität L₁ und einer Kapazität C₁, einem Parallelresonanzschwingkreis PRK mit einer Kapazität Cₚ und einer Induktivität Lₚ, einem Serienresonanzschwingkreis SRK mit einer Induktivität L₂ und Kapazität C₂ und einer Last R_{L} am Ausgang C, D.

Diese Schaltungstopologie ist zwar für eine Spannungsspeisung geeignet, sie besitzt jedoch den Nachteil, dass sich für große Lastwiderstände die Serienresonanz aufspaltet und dadurch stark verschiebt, wie dies aus Fig. 5b ersichtlich ist, da der Eingangs-SRK und PRK miteinander interagieren und die zugehörigen Eingangswiderstände dabei derart kleine Werte annehmen, dass ungünstig große Netzteilströme nötig wären, um die gewünschte Ausgangsleistung zu erzielen. Um dies zu vermeiden ist eine Umschaltung auf Parallelresonanz nötig. Dies führt jedoch zu einem Betriebsmodus mit nichtsinusförmigem Eingangsstrom, der die EMV-Eigenschaften der Anordnung negativ beeinträchtigen würde.

Wie obenstehend erläutert, bestehen also die aus dem Stand der Technik bekannten Maßnahmen zur Minimierung der genannten Nachteile bislang darin, die Schaltungsanordnung eines HF-Generators je nach Anwendungsfall entweder mit einer Strom- oder einer Spannungsspeisung zu versehen. Die schaltungstechnische Realisierung dazu ist jedoch oftmals aufwändig.

WO96/39085A1 offenbart den Oberbegriff des Anspruchs 1.

Der vorliegenden Erfindung liegt deshalb die Aufgabe zugrunde, einen HF-Generator zur Erzeugung von Ausgangsleistungen derart weiterzubilden, dass die aus dem Stand der Technik bekannten und oben diskutierten Probleme und Nachteile vermindert und insbesondere eine hohe HF-Leistungsabgabe mit hohem Wirkungsgrad in einem möglichst großen Lastwiderstandsbereich bei gleichzeitig guten EMV-Eigenschaften erreicht werden kann.

Diese Aufgabe wird erfindungsgemäß durch einen HF-Generator der eingangs genannten Art dadurch gelöst, dass bei dem parallel zum Eingang (A, B) eine Induktivität (Lp) und parallel zum Ausgang (C, D) eine Kapazität (Cp) geschaltet sind, so dass der HF-Generator über einen breiten Lastwiderstandsbereich in Resonanz betreibbar ist.

Ein wesentlicher Punkt der Erfindung liegt darin, dass durch die parallele Induktivität am Eingang die Wirkung eines sonst üblicherweise an ihrer Stelle eingesetzten Parallelresonanzkreises (PRK) und damit die Aufspaltung der Resonanzen bei einer bestimmten Betriebsfrequenz verhindert wird und dass die Kapazität, die parallel zum Ausgang geschaltet ist, eine Serienresonanz auch über einen breiten Lastwiderstandsbereich gewährleistet.

In einer besonderen Realisierung des HF-Generators ist es vorgesehen, dass die Ausgangskapazität Cₚ eine Kapazität aufweist, die betragsmäßig groß ist gegenüber der Kapazität eines Kondensators C₂ des Serienresonanz-Schaltkreises. Diese Dimensionierung erlaubt es, dass die Generatorfrequenz nur eine geringe Abhängigkeit von dem Lastwiderstand aufweist. Aufgrund der betragsmäßigen Größe der Ausgangskapazität Cₚ gegenüber in der Praxis auftretenden kapazitiven Lasten, wie sie beispielsweise durch Ausgangsleitungen oder Endoskope verursacht werden, tritt insbesondere auch keine wesentliche Abhängigkeit der HF-Generatoreigenschaften bei unterschiedlicher kapazitiver Belastung auf.

Ein weiterer wesentlicher Punkt der Erfindung liegt darin, dass die erste Stufe eine Rechteckspannung erzeugt, deren Phasenlage mit einem in den Eingang (A, B) der zweiten Stufe hineinfließenden Eingangsstrom synchronisiert ist. Dadurch wird erreicht, dass der HF-Generator im gewählten Lastwiderstandsbereich in Resonanz betrieben werden kann und ein Nullspannungs- und Nullstromschalten (ZVS und ZCS) der Leistungshalbleiter durch eine minimale induktive Verstimmung gewährleistet wird. Die Phasensynchronisation kann dabei auf einfache Weise mit einer PLL-Schaltung realisiert werden.

Als vorteilhaft für den erfindungsgemäßen HF-Generator ist es, wenn der HF-Chirurgiegenerator einen Spannungsregler zur Überwachung einer Ausgangsspannung U_{A} am Ausgang (C, D) der zweiten Stufe aufweist. Denn insbesondere für große Lastwiderstände steigt die maximale Ausgangspannung stark an, was durch eine entsprechende Schaltungsanordnung verhindert werden muss. Eine, jedoch nicht darauf beschränkte Möglichkeit besteht darin, die Netzteilspannung entsprechend zu reduzieren. Der Spannungsregler kann also zur Regelung eines Netzteils verwendet werden, welches die erste Stufe versorgt.

In einer weiteren vorteilhaften Ausführungsform des HF-Generators ist es darüber hinaus vorgesehen, dass eine Überspannungs-Schutzeinrichtung, die als Varistor ausgebildet sein kann, zwischen dem Verknüpfungspunkt von L₂ und C₂ sowie der Patientenmasse (D bzw. B) eingefügt wird. Auf diese Weise wird dafür Sorge getragen, dass bei einem plötzlichen Lastabfall zum Schutz vor Überspannung die Ausgangsspannung U_{A} bis zum Wirksamwerden eines Reglereingriffs auf unkritische Werte begrenzt wird.

Als besonders bevorzugte Weiterbildung der Erfindung ist ein chirurgisches System mit einem HF-Chirurgiegenerator mit einem chirurgischen Gerät zur Behandlung von Gewebe vorgesehen, das eine Last (R_{L}) mit kapazitivem Anteil darstellt, die am Ausgang (C, D) der zweiten Stufe anliegt. Dabei ist die Kapazität des Ausgangskondensators (Cp) betragsmäßig groß gegenüber dem kapazitiven Anteil der Last (R_{L}). Durch eine derartige Dimensionierung wird sichergestellt, dass das chirurgische System mit dem HF-Chirurgiegenerator über einen breiten Lastwiderstandsbereich in Resonanz betrieben werden kann. Zudem wird dadurch eine Verringerung der Abhängigkeit der Generatoreigenschaften bei unterschiedlicher kapazitiver Belastung erreicht.

Bevorzugte Weiterbildungen der Erfindung sind in den Unteransprüchen angegeben.

Bevorzugte Ausführungsformen der Erfindung werden nachfolgend anhand von Zeichnungen näher beschrieben.

Hierbei zeigen:
Fig. 1a - 1c eine Topologie eines Resonanzkreises zur HF-Leistungserzeugung und den dazugehörigen Verläufen verschiedener Ausgangsgrößen gemäß der vorliegenden Erfindung;
Fig. 2 eine detaillierte Schaltung einer Ausführungsform der vorliegenden Erfindung;
Fig. 3a - 3b einen Resonanzfrequenzverlauf und die Ausgangscharakteristik des erfindungsgemäßen HF-Generators gemäß Fig. 2.
Fig. 4a - 4c eine aus dem Stand der Technik bekannte Topologie eines Resonanzkreises zur HF-Leistungserzeugung mit einem Eingangs-Parallelresonanzschwingkreis mit den dazugehörigen Verläufen verschiedener Ausgangsgrößen; und
Fig. 5a - 5c eine aus dem Stand der Technik bekannte Topologie eines Resonanzkreises zur HF-Leistungserzeugung mit einem Eingangs-Serienresonanzschwingkreis mit den dazugehörigen Verläufen verschiedener Ausgangsgrößen;

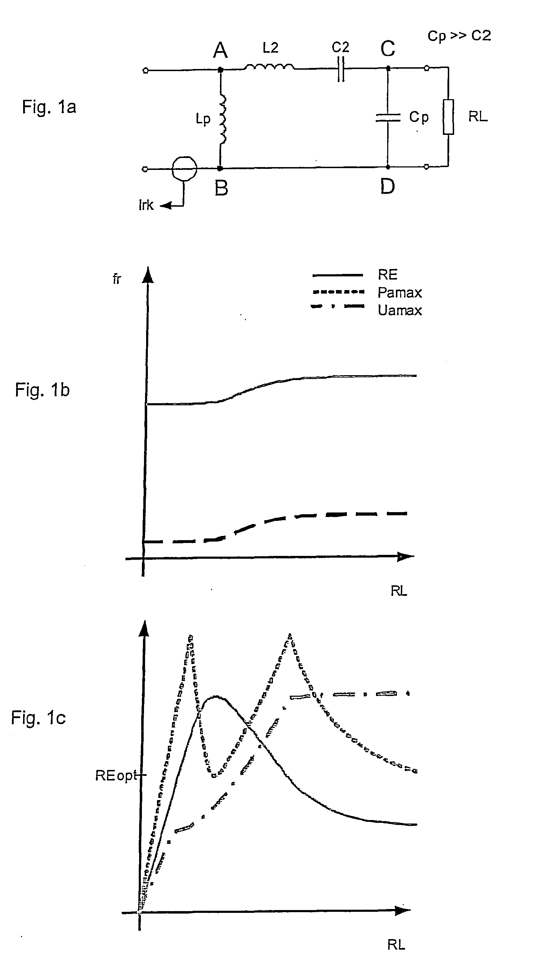

Fig. 1a zeigt einen Resonanzkreis gemäß der vorliegenden Erfindung mit einer am Eingang A, B parallel liegenden Induktivität Lₚ, einem Serienresonanzschwingkreis SRK mit einer Induktivität L₂ und Kapazität C₂ und einer am Ausgang C, D parallel liegenden Kapazität Cₚ und einer Last R_{L}.

Die Nachteile der in Fig. 4a und Fig. 5a beschriebenen und aus dem Stand der Technik bekannten Topologien lassen sich mit der Topologie gemäß Fig. 1a vermeiden, indem die Wirkung eines PRK durch die Parallelinduktivität Lp ersetzt und damit die Aufspaltung der Resonanzen verhindert wird. Durch das zusätzliche Hinzufügen der Kapazität Cₚ wird ein Betrieb der Schaltung in Serienresonanz auch für große Lastwiderstände R_{L} gewährleistet. Ein Eingangs-SRK ist dann nicht mehr zwingend nötig. Die Serienresonanzkapazität C₂ kann teilweise und die Serienresonanzinduktivität L₂ kann auch ganz unter Berücksichtigung des Übersetzungsverhältnisses bei einem vorhandenen Transformator auf dessen Primärseite verlagert werden. Die durch die Verlagerung der Serienblindelemente C₂ und L₂ auf die Primärseite eines Transformators entstandenen Topologien zeigen prinzipiell keine anderen Eigenschaften und sind im Sinne der Erfindung als gleichwirkend anzusehen.

Die in der Fig. 1a dargestellte Topologie weist eine durchgängige Serienresonanz auf, deren Lastabhängigkeit gering gehalten werden kann, wenn Cₚ>>C₂ gewählt wird. Diese Ausführungsform des Resonanzkreises kann beispielweise durch eine mit einer Halb- oder Vollbrücke erzeugten Rechteckspannung, deren Phasenlage mit dem Filtereingangsstrom synchronisiert ist, im gesamten Lastwiderstandsbereich in Resonanz betrieben werden. Durch eine minimal induktive Verstimmung wird Nullspannungs- und Nullstromschalten (ZVS und ZCS) der Leistungshalbleiter gewährleistet. Die Phasensynchronisation kann auf einfache Weise mit einer PLL-Schaltung realisiert werden.

Erfindungsgemäß wird eine direkte Rückkopplung des Stromsignals auf die Ansteuerung des Resonanzbetriebs gewährleistet. Durch symmetrische, kontinuierliche Ansteuerung wird ein Ausgangssignal mit einem sehr geringen Klirrgrad erzeugt. Dies erlaubt die Auswertung von Oberwellen als Messgröße für Gewebeeigenschaften und zur Sicherung einer hohen Prozeßqualität.

Darüber hinaus lassen sich durch geeignete Dimensionierung hohe Ausgangsleistungen speziell bei kleinen Lastwiderständen und damit hohe Ausgangsströme erzielen, wie sie etwa für Verfahren in der Urologie und zur bipolaren Gefäßkoagulation benötigt werden. Gleichzeitig lässt sich für den gesamten relevanten Lastwiderstandsbereich, insbesondere für den oberen Bereich, ein relativ großer Filtereingangswiderstand realisieren. Dadurch sind keine allzu großen Netzteilströme erforderlich, was eine wesentliche Voraussetzung für den hohen Wirkungsgrad ist.

Günstiges EMV-Verhalten des HF-Generators kann dadurch gewährleistet werden, da Nullspannungs- und Nullstromschalten (ZCS und ZVS-Betrieb) der Leistungshalbleiter möglich ist und sich im gesamten Betriebsbereich ein sinusförmiger Eingangsstrom einstellt.

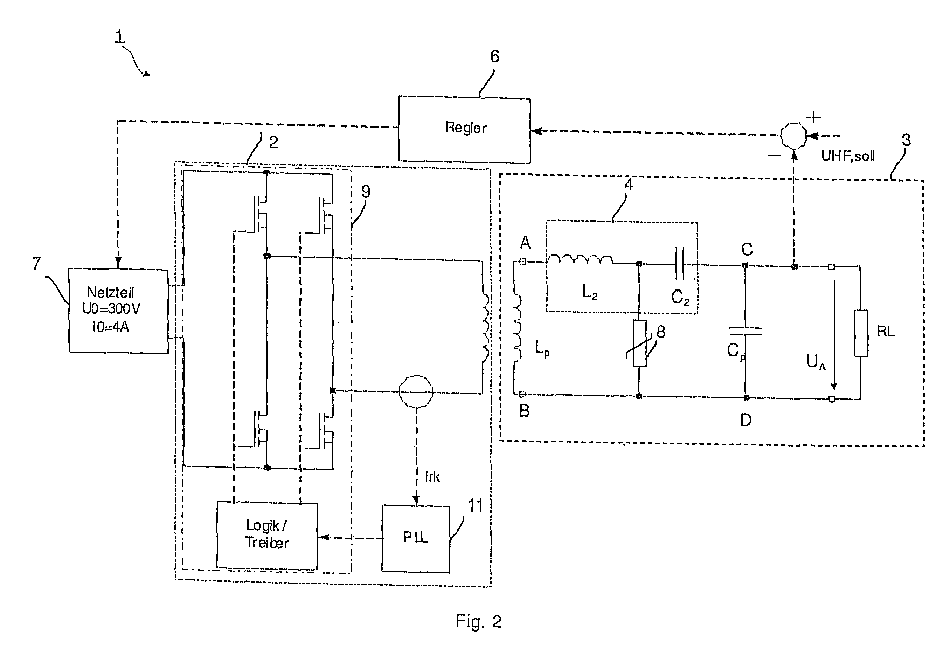

Fig. 2 zeigt ein Ausführungsbeispiel für einen erfindungsgemäßen HF-Generator. Dieser besteht aus einem Netzteil 7 und einer ersten Stufe 2, die mit einer zweiten Stufe 3 gekoppelt ist. Die erste Stufe 2 weist im dargestellten Ausführungsbeispiel eine Treiberstufe 9, die als Vollbrücke ausgebildet ist und eine Rechteckspannung erzeugt und eine PLL-Schaltung 11 zur Phasensynchronisation der von der Treiberstufe erzeugten Rechteckspannung mit einem Eingangsstrom Iᵣₖ auf. Die zweite Stufe 3 weist einen Eingang A, B und einen Ausgang C, D auf, wobei sich zwischen dem Eingang A, B und dem Ausgang C, D der zweiten Stufe 3 ein Serienresonanz-Schaltkreis 4 mit einer Induktivität L₂ (200 µH) und einer Kapazität C₂ (1 nF) befindet. Parallel zum Eingang A, B liegt eine Induktivität Lp (196 µH) und parallel zum Ausgang C, D sind eine Kapazität Cₚ (4 nF) angeordnet und eine Last R_{L} geschaltet. Zwischen dem Verknüpfungspunkt von L₂ und C₂ sowie dem Ausgang D befindet sich eine Überspannungsschutzeinrichtung 8 mit einem Überspannungsschutz für Spannungen bis zu 1 kV, die beispielsweise als Varistor ausgebildet sein kann.

Über einen Spannungsregler 6 wird die Ausgangsspannung U_{A} am Ausgang C, D überwacht. Bei einem plötzlichen Lastabfall begrenzt zunächst der Überspannungsschutz 8 die Ausgangsspannung U_{A} bis zum Wirksamwerden eines Reglereingriffs auf unkritische Werte. Die gezeigte Ausführungsform sieht vor, dass der Spannungsregler 6 über die rückgekoppelte Ausgangsspannung U_{A} das Netzteil 7 regelt, welches die erste Stufe 2 versorgt.

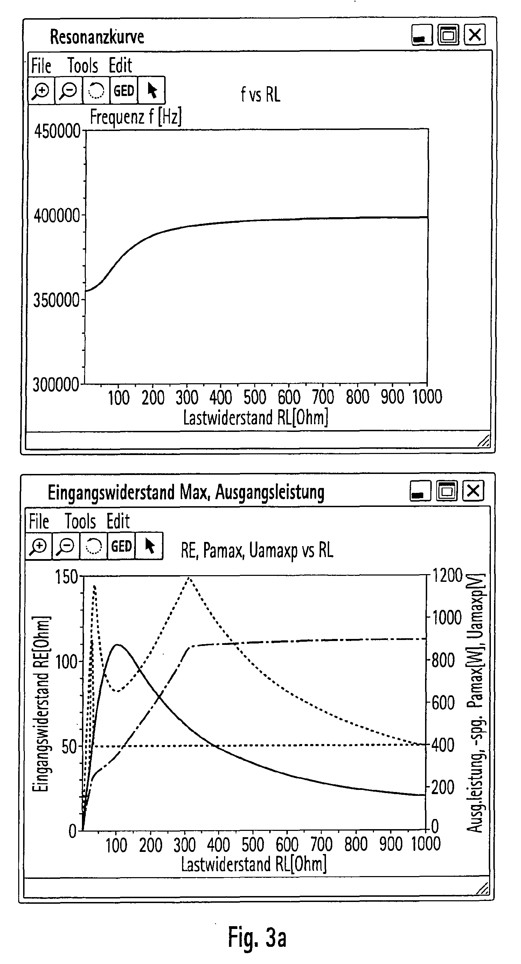

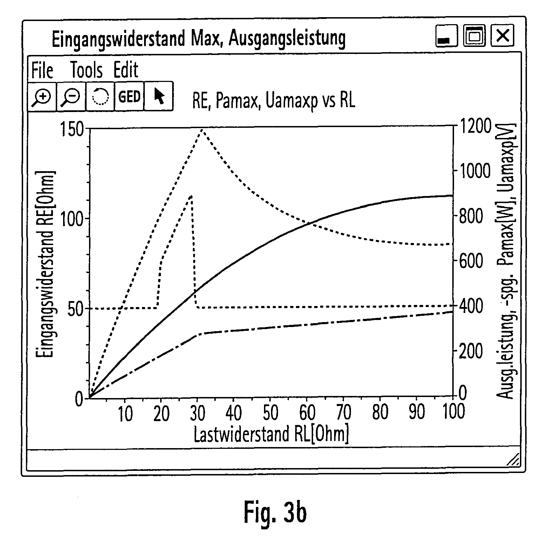

Fig. 3a und Fig. 3b zeigen jeweils den Resonanzfrequenzverlauf und die Ausgangscharakteristik des in Fig. 2 dargestellten HF-Generators. Die Parameter des für die Ausgangscharakteristik zugrunde gelegten Netzteils sind U₀=300V und I₀=4A. Der optimale Filtereingangswiderstand für max. Ausgangsleistung ergibt sich zu: REₒₚₜ=60.8Ω.

Da die Kapazität Cₚ am Ausgang C, D groß gegenüber der Kapazität C₂ des Serienresonanzkreises gewählt ist, tritt nur eine relativ geringe Abhängigkeit der Generatorfrequenz vom Lastwiderstand auf. Dadurch, dass die Kapazität Cₚ groß gegenüber in der Praxis (durch Ausgangsleitungen oder Endoskope) auftretenden kapazitiven Lasten gewählt ist, tritt insbesondere auch keine wesentliche Abhängigkeit der Generatoreigenschaften bei unterschiedlicher kapazitiver Belastung auf. Das ist günstig für die Auslegung einer PLL-Schaltung und vereinfacht die Messung und Auswertung der Ausgangssignale. Mit unterbrochener Linie ist in Fig. 3a ein Toleranzschema für die Ausgangsleistung eingetragen, wie es für einen Generator für die bipolare TUR angestrebt wird. In der gespreizten Darstellung des unteren Lastwiderstandsbereichs der Fig. 3b kann man erkennen, dass das Toleranzschema erfüllt wird.

### Bezugszeichenliste

- 1: HF-Chirurgiegenerator
- 2: erste Stufe des HF-Chirurgiegenerators
- 3: zweite Stufe des HF-Chirurgiegenerators
- 4: Serienresonanz-Schaltkreis
- 5: Eingangsstrom
- 6: Spannungsregler
- 7: Netzteil
- 8: Überspannungs-Schutzeinrichtung
- 9: Treiberstufe
- 11: PLL-Schaltung
- A, B: Eingang der zweiten Stufe
- C, D: Ausgang der zweiten Stufe
- Cp: Ausgangskapazität
- C2: Kapazität des Serienresonanzkreises
- Ik: Eingangsstrom
- Lp: Induktivität
- L2: Induktivität
- RL: Lastwiderstand
- UA: Ausgangsspannung der zweiten Stufe

## Patentansprüche

1. HF-Chirurgiegenerator (1) zur Erzeugung von Ausgangsleistungen mit hohem Wirkungsgrad, mit einer ersten Stufe (2) zur Erzeugung einer HF-Leistung, die mit einer zweiten Stufe (3) gekoppelt ist, wobei die zweite Stufe (3) einen Eingang (A, B) und einen Ausgang (C, D) aufweist, und wobei sich zwischen dem Eingang (A, B) und dem Ausgang (C, D) der zweiten Stufe (3) ein Serienresonanz-Schaltkreis (4) befindet, wobei
parallel zum Eingang (A, B) eine Induktivität (Lp) und parallel zum Ausgang (C, D) eine Kapazität (Cp) geschaltet sind, so dass der HF-Generator (1) über einen breiten Lastwiderstandsbereich in Resonanz betreibbar ist,
**dadurch gekennzeichnet,**
**dass** die erste Stufe (2) eine Rechteckspannung erzeugt, deren Phasenlage mit einem in den Eingang (A, B) der zweiten Stufe (3) hineinfließenden Eingangsstrom (5) synchronisiert ist.

2. HF-Chirurgiegenerator nach Anspruch 1,
**dadurch gekennzeichnet, dass**
die Ausgangskapazität (Cp) eine Kapazität aufweist, die betragsmäßig groß ist gegenüber der Kapazität eines Kondensators (C₂) des Serienresonanz-Schaltkreises (4).

3. HF-Chirurgiegenerator nach einem der vorhergehenden Ansprüche,
**dadurch gekennzeichnet, dass**
der HF-Chirurgiegenerator (1) einen Spannungsregler (6) zur Überwachung einer Ausgangsspannung U_{A} am Ausgang (C, D) der zweiten Stufe (3) aufweist.

4. HF-Chirurgiegenerator nach einem der vorhergehenden Ansprüche,
**dadurch gekennzeichnet, dass**
der Spannungsregler (6) zur Regelung eines Netzteilgerätes (7) ausgebildet ist, welches die erste Stufe (2) versorgt.

5. HF-Chirurgiegenerator nach einem der vorhergehenden Ansprüche,
**dadurch gekennzeichnet, dass**
eine Überspannungs-Schutzeinrichtung, insbesondere ein Varistor (8), innerhalb der zweiten Stufe (3) vorgesehen ist, so dass zum Schutz vor Überspannung die Ausgangsspannung U_{A} bei einem plötzlichem Lastabfall begrenzt wird.

6. Chirurgisches System mit einem HF-Chirurgiegenerator (1) nach einem der vorhergehenden Ansprüche und mit einem chirurgischen Gerät zur Behandlung von Gewebe, das eine Last (R_{L}) mit kapazitivem Anteil darstellt, die am Ausgang (C, D) der zweiten Stufe (3) anliegt, **dadurch gekennzeichnet, dass** die Kapazität des Ausgangskondensators (Cp) betragsmäßig groß gegenüber dem kapazitiven Anteil der Last (R_{L}) ist.

## Claims

1. RF surgical generator (1) for generating output powers with high efficiency, having a first stage (2) for generating an RF power which is coupled to a second stage (3), the second stage (3) having an input (A, B) and an output (C, D), and a series resonant circuit (4) being situated between the input (A, B) and the output (C, D) of the second stage (3), an inductance (Lₚ) being connected in parallel with the input (A, B) and a capacitance (Cp) being connected in parallel with the output (C, D), with the result that the RF generator (1) can be operated at resonance over a wide load resistance range, **characterized in that** the first stage (2) generates a square-wave voltage, the phase angle of which is synchronized with an input current (5) flowing into the input (A, B) of the second stage (3).

2. RF surgical generator according to Claim 1, **characterized in that**
the output capacitance (Cₚ) has a capacitance, the magnitude of which is large in comparison with the capacitance of a capacitor (C₂) in the series resonant circuit (4).

3. RF surgical generator according to one of the preceding claims,
**characterized in that**
the RF surgical generator (1) has a voltage regulator (6) for monitoring an output voltage U_{A} at the output (C, D) of the second stage (3).

4. RF surgical generator according to one of the preceding claims,
**characterized in that**
the voltage regulator (6) is designed to regulate a power supply device (7) which supplies the first stage (2).

5. RF surgical generator according to one of the preceding claims,
**characterized in that**
an overvoltage protection device, in particular a varistor (8), is provided inside the second stage (3), with the result that the output voltage U_{A} is limited in the event of a sudden load drop in order to protect against overvoltage.

6. Surgical system having an RF surgical generator (1) according to one of the preceding claims and having a surgical device for handling tissue which constitutes a load (R_{L}) with a capacitive portion, which load is present at the output (C, D) of the second stage (3), **characterized in that** the magnitude of the capacitance of the output capacitor (Cₚ) is large in comparison with the capacitive portion of the load (R_{L}).

## Revendications

1. Générateur chirurgical HF (1) pour la génération de puissances de sortie à rendement élevé, avec un premier étage (2) pour la génération d'une puissance HF, lequel est couplé avec un deuxième étage (3), dans lequel le deuxième étage (3) présente une entrée (A, B) et une sortie (C, D), et dans lequel un circuit de commutation résonnant en série (4) se trouve entre l'entrée (A, B) et la sortie (C, D) du deuxième étage (3),
dans lequel une inductance (Lp) est couplée parallèlement à l'entrée (A, B) et une capacité (Cₚ) est couplée parallèlement à la sortie (C, D) de telle sorte que le générateur HF (1) peut être exploité en résonnance sur une plage étendue de résistances de charge,
**caractérisé en ce que** le premier étage (2) génère une tension rectangulaire dont la position de phase est synchronisée avec un courant d'entrée (5) circulant à l'intérieur de l'entrée (A, B) du deuxième étage (3).

2. Générateur chirurgical HF selon la revendication 1,
**caractérisé en ce que** la capacité de sortie (Cp) présente une capacité qui est d'un montant important par rapport à la capacité d'un concentrateur (C₂) du circuit de commutation résonnant en série (4).

3. Générateur chirurgical HF selon l'une des revendications précédentes,
**caractérisé en ce que** le générateur chirurgical HF (1) présente un régulateur de tension (6) pour la surveillance d'une tension de sortie U_{A} à la sortie (C, D) du deuxième étage (3).

4. Générateur chirurgical HF selon l'une des revendications précédentes,
**caractérisé en ce que** le régulateur de tension (6) est conçu pour la régulation d'un appareil d'alimentation électrique (7) qui alimente le premier étage (2).

5. Générateur chirurgical HF selon l'une des revendications précédentes,
**caractérisé en ce que** l'on prévoit un dispositif de protection contre les surtensions, en particulier une varistance (8), à l'intérieur du deuxième étage (3) de telle sorte que, pour la protection contre les surtensions, la tension de sortie U_{A} est limitée en cas de chute soudaine de charge.

6. Système chirurgical avec un générateur chirurgical HF (1) selon l'une des revendications précédentes et avec un appareil chirurgical pour le traitement de tissus, lequel constitue une charge (R_{L}) avec une part capacitive, laquelle est appliquée à la sortie (C, D) du deuxième étage (3),
**caractérisé en ce que** la capacité du condensateur de sortie (Cp) est d'un montant important par rapport à la part capacitive de la charge (R_{L}).
